Europäisches Patentamt

European Patent Office

Office européen des brevets

(19)

(11) Publication number: **0 141 636**
A1

## (12) EUROPEAN PATENT APPLICATION

(21) Application number: **84307437.8**

(22) Date of filing: **29.10.84**

(51) Int. Cl.⁴: **G 01 N 33/28**, G 01 N 27/22

(30) Priority: **04.11.83 GB 8329483**

(43) Date of publication of application: **15.05.85
Bulletin 85/20**

(84) Designated Contracting States: **DE FR GB**

(71) Applicant: **ANDERSON STRATHCLYDE PLC, Anderson
House 47 Broad Street, Glasgow, G40 2QW Scotland
(GB)**

(72) Inventor: **Mackie, Kenmuir, 14 Kirk Street, Carluke (GB)**
Inventor: **Schleyer, Graham Klaus, 12 Marigold Way,
Carluke (GB)**
Inventor: **Thomas, Robert Victor, Trotwood Cottage
Gaunts Common, Wimborne Dorset (GB)**

(74) Representative: **Wotherspoon, Graham et al,
FITZPATRICKS 4 West Regent Street, Glasgow G2 1RS
Scotland (GB)**

(54) **Detection of water in oil.**

(57) The invention provides a capacitive transducer through which oil, e.g. lubricating oil in a mining machine, flows. The transducer comprises a cylindrical casing 10 in which end members 22, 24 locate coaxial cylinders 16, 18 forming capacitor electrodes. Preferably, the outer electrode 16 is electrically connected to a central tie bolt 26.

- 1 -

"Detection of water in oil"

This invention relates to a method of and apparatus for detecting water in oil, for example in the lubricating oil of an engine or gearbox or in hydraulic oil. The invention is envisaged as being particularly, but not exclusively, applicable in mining machinery.

Mining machinery operates in extremely arduous conditions, and is particularly susceptible to ingress of water into gearboxes and hydraulic systems which are operated near the operating limits of the oils used. Ingress of relatively small concentrations of water can cause a substantial reduction in lubricity, leading to an expensive failure. Hitherto, this problem has been dealt with by frequent oil changes in a preventive maintenance schedule.

An object of the present invention is to provide a simple, reliable and rugged means of monitoring water concentration in oil on a continuous or sampling basis.

The invention accordingly provides a transducer for detecting water in oil, comprising a casing having connection means for connecting the transducer in an oil line to allow continuous flow of oil through the casing, and at least two spaced electrodes positioned within the casing such that oil flowing therethrough acts as a dielectric medium whereby the capacitance between the electrodes is a function of the concentration of water in the oil.

From another aspect, the invention provides a method of detecting water in oil in a machine having a circuit around which oil is continuously pumped, the method comprising continuously monitoring the capacitance of the oil at a given point in said circuit.

An embodiment of the invention will now be described, by way of example, with reference to the accompanying drawings, in which:

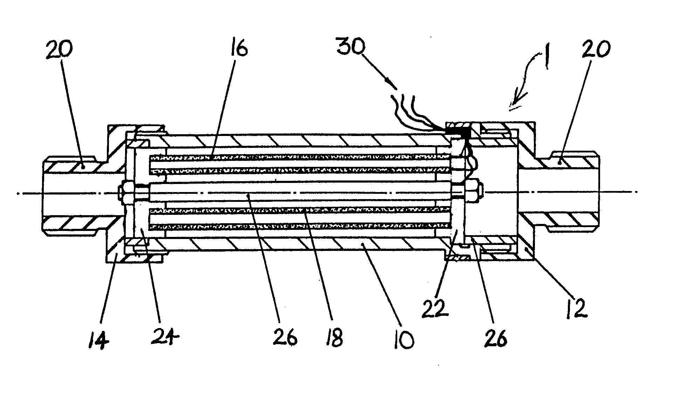

Fig. 1 is a cross-sectional elevation of a transducer embodying the invention;

Figs. 2 and 3 illustrate circuitry for use with the transducer;

Figs. 4A and 4B schematically illustrate the electrical and mechanical connections, respectively of the transducer in a temperature-compensated arrangement; and

Fig. 5 illustrates the transducer operating in conjunction with a microprocessor, with temperature compensation.

The transducer 1 comprises a cylindrical casing 10 of stainless steel with its ends threaded to receive end caps 12,14 also of stainless steel. The end caps 12,14 are provided with 3/4" BSPP fittings 20 whereby the transducer can be connected in an existing oil circulation line of, for example, a mining machine gearbox.

Within the casing 10 a capacitor formed by concentric cylinders 16 and 18 is located by means of centralisers 22,24 held together by a central support and tie bar 26. Spacer ring 26 maintains a correct spacing between the end plate 22 and end cap 12. The cylinders 16,18 and tie bar 26 are provided with electrical leads 30 exiting through a suitable seal. In use, the outer cylinder 16 and the tie bar 26 are electrically connected together (for maximum reference capacitance values) to form one electrode of a capacitor, the inner cylinder 18 forming the other electrode. The cylinders 16 and 18 are suitably of stainless steel coated in a plastic such a Parylene C for electrical insulation from each other. This coating is applied using high vacuum chambers. The plastic is vapourised and the assembly is coated completely with a thin, even laminate.

During assembly, the concentric cylinders 16,18, and plates 22,24 and tie bar 26 are pre-assembled and inserted into the casing from one end. This has an advantage in that the wiring coupling may be carried out outside the complete transducer assembly making the insulation easier to apply. Furthermore, the spacer ring 26 is designed to permit the free end of the assembly to move within the casing due to thermal expansion.

In use, the transducer is connected, for example, in the oil line of a mining machine gearbox. The gearbox oil passes continuously through the transducer and its capacitance is continuously monitored. It has been found that readily detectable capacitance differences are caused by the presence of small percentages of water in oil. For example, Table 1 gives capacitance values for water in "Tellus 100' oil at 50°C with a prototype transducer.

TABLE 1

| % water | Capacitance (pF) |
|---------|------------------|
| 0       | 84               |
| 1       | 87               |
| 2       | 92               |
| 4       | 96               |
| 5       | 99               |
| 6       | 101              |
| 8       | 104              |
| 10      | 107              |

Thus a small concentration can be detected and an alarm given to initiate remedial action.

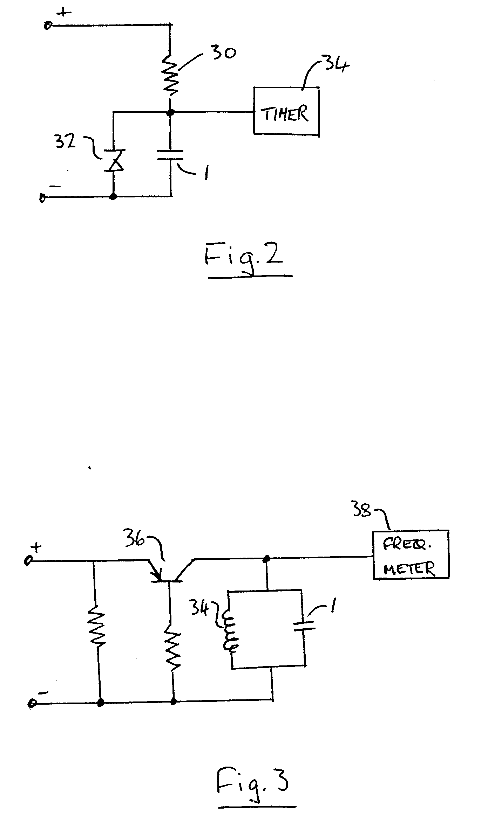

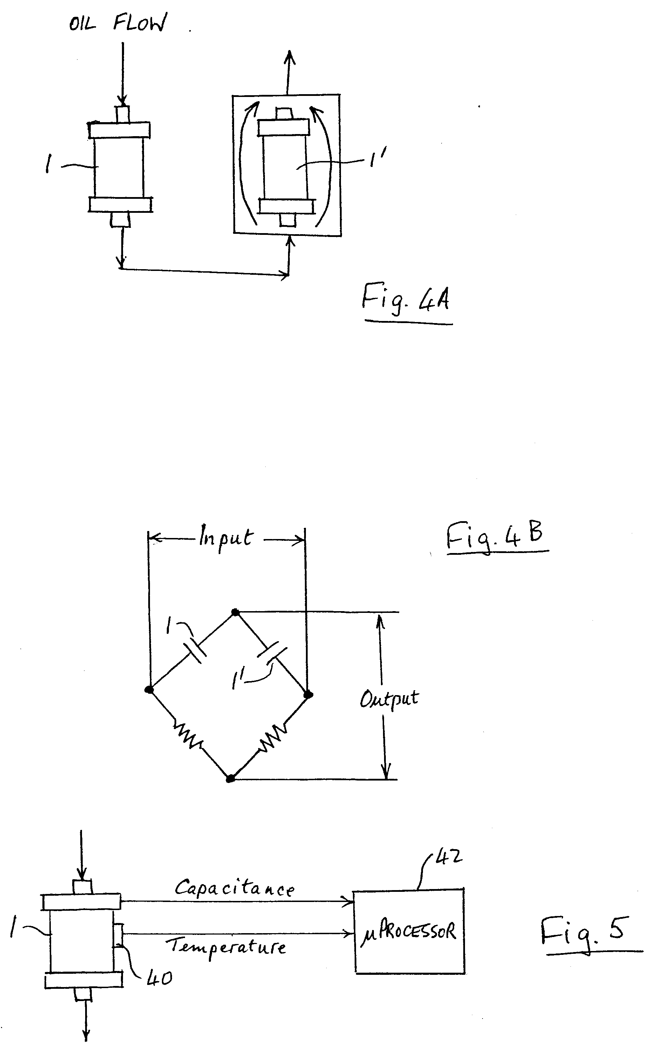

Various types of electronic circuitry for use with the transducer in measuring its capacitance will readily be apparent. For example, as shown in Fig. 2, the transducer 1 may be connected with a resistor 30 and Zener diode 32 to form a RC charge-dischrage circuit, the capacitance being determined by a timer 34 measuring the charging time.  Alternatively, as shown in Fig. 3 the transducer 1 may form part of an oscillator in which the transducer 1 and an inductor 34 form a feedback path for transistor 36; the capacitance can be determined by measuring the oscillator frequency by meter 38.

The capacitance of the transducer varies with the temperature of the oil, and means may therefore be provided to compensate for this.

In Fig. 4, a second transducer 1' is filled with water-free oil and sealed.  The circulating oil passes around the second transducer 1' to maintain it at the same temperature as the transducer 1.  The transducers 1,1' are electrically connected in a bridge circuit as shown in Fig. 4B so that temperature effects cancel.

Alternatively, the transducer may form part of a microprocessor-based monitoring system.  In this case, temperature variation may be compensated by calculation using stored data defining the characteristics of capacitance versus temperature.  This is achieved, as in Fig. 5, by mounting a semiconductor temperature sensor 40 on the transducer 1.  The output signal of the sensor 40 together with the capacitance signal fed to the microprocessor 42 where they are equated electronically to shift the co-ordinates of the calibration characteristics yielding the value of percentate water contamination.

CLAIMS

1.    A transducer for detecting water in oil, comprising a casing having connection means for connecting the transducer in an oil line to allow continuous flow of oil through the casing, and at least two spaced electrodes positioned within the casing such that oil flowing therethrough acts as a dielectric medium whereby the capacitance between the electrodes is a function of the concentration of water in the oil.

2.    A transducer according to claim 1, in which the casing is cylindrical with said connection means at opposite ends, and the electrodes comprise concentric tubes mounted within the casing.

3.    A transducer according to claim 2, in which the ends of the tubes are held in spacers which are interconnected by a central axial tie bar, the outer tube and the tie bar being electrically interconnected to form a single electrode.

4.    Apparatus for detecting oil in water, comprising at least one transducer in accordance with any preceding claim, and electronic circuitry connected to the transducer to supply electrical excitation and monitor said capacitance.

5.    Apparatus according to claim 4, including means to compensate for temperature variation in the oil being monitored.

6.    Apparatus according to claim 5, in which said temperature compensating means comprises a second transducer filled with a reference oil, the two transducers being connected in a bridge circuit.

7.    Apparatus according to claim 5, in which said temperature compensating means comprises an oil temperature detector, a memory holding data representing the variation of capacitance of water-free oil with temperature, and calculating means arranged to normalise the measured capacitance in accordance with the measured temperature.

8.    A method of detecting water in oil in a machine having a circuit around which oil is continuously pumped, the method comprising continuously monitoring the capacitance of the oil at a given point in said circuit.

9.    The method of claim 8, in which the temperature of the oil is also measured and the measured capacitance modified in dependence on temperature.

1/3

Fig.2

Fig. 3

OIL   FLOW

Fig. 4A

Fig. 4 B

Input

Output

Capacitance

μPROCESSOR

Temperature

42

40

Fig. 5

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | US-A-3 546 926 (S.W. DUNAVENT) <br><br> * Whole document * | 1,4-6, 9 | G 01 N 33/28 <br> G 01 N 27/22 |
| Y | EP-A-0 072 053 (SHELL INTERNATIONALE RESEARCH) <br> * Abstract; claims; figure 1 * | 1,2,4 | |
| A | EP-A-0 070 072 (SHELL INTERNATIONALE RESEARCH) <br> * Claims * | 1,4,5, 9 | |
| A | EP-A-0 080 755 (MOTOROLA INC.) <br> * Claims * | 1,4,6 | |
| A | EP-A-0 019 154 (BASF AG) <br> * Claims * | 1,4,7 | TECHNICAL FIELDS SEARCHED (Int. Cl.4) |
| A | FR-A-2 251 241 (C.E.D.E.M.) <br> * Whole document * | 1,4,7 | G 01 N 33/28 <br> G 01 N 27/22 |

The present search report has been drawn up for all claims

| Place of search <br> THE HAGUE | Date of completion of the search <br> 08-02-1985 | Examiner <br> CALLEWAERT-HAEZEBROU |
|---|---|---|